# EUROPÄISCHE PATENTANMELDUNG

(11) **EP 3 108 809 A1**
(43) Veröffentlichungstag der Anmeldung: **28.12.2016**
(21) Anmeldenummer: 15173069.4
(22) Anmeldetag: 22.06.2015
(51) Int. Cl.: A61B 5/0215, A61B 5/07, A61B 5/00

(54) **VORRICHTUNG UND VERFAHREN ZUR DRUCKMESSUNG AM HERZEN EINES PATIENTEN**

(71) Anmelder: Berlin Heart GmbH, 12247 Berlin (DE)
(72) Erfinder: Granegger, Marcus, 10963 Berlin (DE)
(74) Vertreter: Pfenning, Meinig & Partner mbB

(57) **Zusammenfassung**

Es wird eine Vorrichtung zur Druckmessung am Herzen eines Patienten vorgestellt, mit einer Sensoreinrichtung (13, 14, 15, 21, 22, 25, 27, 32, 34, 35, 44, 45, 48, 49, 50, 51, 67), die die Druckdifferenz zwischen einer Stelle innerhalb des Herzens (2) einerseits und dem außerhalb des Herzens innerhalb des Thorax (8) liegenden Raum andererseits erfasst, wobei beispielsweise ein Element der Sensoreinrichtung, beispielsweise ein Sensor, an einem mit dem Herzen verbundenen Gerät, beispielsweise einer Herzpumpe (3, 3' 55) oder einem Herzschrittmacher (17), angeordnet ist.

## Beschreibung

Die Erfindung liegt auf dem Gebiet der Mechanik und der Elektrotechnik und ist mit besonderem Vorteil auf dem Gebiet der Medizintechnik einsetzbar. Sie befasst sich konkret mit der Druckmessung am Herzen eines Patienten mithilfe von Sensoreinrichtungen.

Es ist eine Vielzahl von Geräten im medizinischen Bereich entwickelt worden, die der Überwachung oder Unterstützung von Patienten dienen und die im Körper des Patienten ganz oder teilweise implantiert werden können. Hierzu zählen beispielsweise Herzschrittmacher, Defibrillatoren und auch Herzunterstützungssysteme in Form von voll oder teilweise implantierten Herzpumpen.

Für einige solcher implantierbarer Geräte ist die Überwachung des Fluiddrucks im Inneren des Herzens, beispielsweise in einem Ventrikel oder Atrium, wichtig.

Für eine physikalische und physiologische Analyse solcher Messdaten ist der Vergleich mit Referenzdrücken bzw. die Berücksichtigung eines Referenzdrucks unabdingbar. Für solche Zwecke wurden daher bisher Absolutdrucksensoren im Herzen in Verbindung mit einer Messung des barometrischen gemessenen Drucks außerhalb des Körpers verwendet. Beispiele solcher Druckmessungen gehen aus den Druckschriften US 2007/0282210 A1, EP 1415 672 B1 sowie US 6 328 699 B1 hervor. Aus der WO 2013/7.22459 A1 ist unabhängig von dem Betrieb eines Geräts ein Diagnosesystem beschrieben, das den Differenzdruck zwischen dem rechten intraatrialen Druck und dem Perikardium misst, um auf eine Herztamponade zurückschließen zu können.

Der vorliegenden Erfindung liegt vor dem Hintergrund des Standes der Technik die Aufgabe zugrunde, eine Vorrichtung zur Druckmessung am Herzen eines Patienten zu schaffen, die die Messung eines korrigierten Drucks in Verbindung mit dem Betrieb eines mit dem Herzen des Patienten verbundenen Geräts in effizienter, möglichst einfacher, kostengünstiger und für den Patienten möglichst wenig belastender Weise erlaubt.

Die Aufgabe wird mit den Merkmalen des Patentanspruchs 1 gelöst. Die Patentansprüche 2 bis 11 nennen konkrete Ausgestaltungen der Lösung. Der Patentanspruch 12 bezieht sich auf ein Verfahren zur Lösung der Aufgabe, der Anspruch 13 auf ein Computerprogrammprodukt und die Ansprüche 14 und 15 auf eine Regeleinrichtung.

Die Aufgabe wird somit gelöst durch eine Vorrichtung zur Druckmessung am Herzen eines Patienten, gekennzeichnet durch eine Sensoreinrichtung, die die Druckdifferenz zwischen einer Stelle innerhalb des Herzens einerseits und dem außerhalb des Herzens innerhalb des Thorax liegenden Raum andererseits erfasst. Dabei kann wenigstens ein Element der Sensoreinrichtung an einem mit dem Herzen verbundenen Gerät, insbesondere einer Herzpumpe, einem Herzschrittmacher oder Defibrillator, angeordnet sein.

Auf den Druck im Inneren eines Herzens haben sowohl der reale Ventrikeldruck, gegeben durch die statischen und dynamischen Verhältnisse im Herzen selbst, als auch der außerhalb des Herzens im Thorax herrschende Druck und der außerhalb des Patientenkörpers auf diesen wirkende barometrische Druck einen Einfluss.

Zur Bestimmung bestimmter physiologischer Größen im Zusammenhang mit der Überwachung eines Herzens ist oft nur der Unterschied zwischen dem absoluten Druck im Inneren des Herzens und dem Perikardium bzw. Thorax wichtig, der auch als transmuraler Druck bezeichnet wird und der ursächlich ist für die auf die Herzwand wirkenden Kräfte. Es ist daher kaum möglich, ausschließlich aus einer Druckmessung im Inneren des Herzens auf die vorliegenden Bedingungen zu schließen. Auch die Berücksichtigung des barometrischen Drucks allein hilft nicht, da beispielsweise beim Ein- und Ausatmen der Thoraxdruck periodisch variiert und somit der Druck im Herzinneren ebenso. Beispielsweise lässt sich bei einer implantierten Herzpumpe, die über einen Herzkatheter im Herzinnenraum Blut absaugt, nur durch eine Messung des Drucks im Herzinnenraum (Ventrikel oder Atrium) kein eindeutiger Rückschluss auf ein Ansaugen der Kanüle an einer Herzwand ziehen, da etwaige Druckschwankungen ebenso durch Druckschwankungen im Thorax hervorgerufen werden können. Der transmurale Druck ist von solchen Effekten weniger betroffen.

Die vorliegende Lösung erlaubt die Messung des transmuralen Drucks und damit eine verbesserte Steuerungsmöglichkeit für das jeweils mit dem Herzen verbundene Gerät, wie beispielsweise eine Herzpumpe, einen Herzschrittmacher oder einen Defibrillator.

Die Sensoreinrichtung kann dazu sowohl mit dem Raum innerhalb des Herzens / der Herzwand als auch mit dem Raum außerhalb des Herzens im Perikardium oder im Thorax verbunden sein. Die Verbindung kann dabei, wie weiter unten näher ausgeführt wird, elektrisch oder hydraulisch (mittels eines Fluidkanals) oder über eine andere Kommunikationsart realisiert sein. Vorteilhaft ist jedenfalls die Verbindung wenigstens eines Teils der Sensoreinrichtung mit einem Gerät, so dass der Aufwand bei der Herstellung und auch bei der Implantierung des Gesamtaggregats optimiert wird. Fehlerquellen beim Betrieb können so minimiert werden, und es kann eine zuverlässige Platzierung im Patientenkörper erreicht werden.

Wenigstens ein Sensor der Sensoreinrichtung und gegebenenfalls auch ein Fluidkanal, mit dem der Sensor verbunden ist oder in dem er angeordnet ist, können mit einem Gerät, wie beispielsweise einer Herzpumpe, fest verbunden oder an diesem befestigt sein.

Eine Ausgestaltung der Lösung kann beispielsweise vorsehen, dass wenigstens ein Element der Sensoreinrichtung an einem mit dem Herzen verbundenen Gerät, insbesondere einer Herzpumpe, angeordnet ist. In diesem Fall kann vorgesehen sein, dass die Sensoreinrichtung nur einen einzigen Drucksensor in Form eines Druckdifferenzsensors aufweist. Dieser kann beispielsweise eine Messmembran aufweisen, wobei auf einer ersten Seite der Membran der Druck vom Herzinneren ansteht, während auf der zweiten Seite der Membran der Druck aus dem Thoraxbereich ansteht. Beispielsweise kann eine derartige Membran oder Sensoreinrichtung unmittelbar eine durchgehende Öffnung in der Herzwand verschließen bzw. innerhalb der Herzwand vorgesehen sein. Ein derartiger Differenzdrucksensor kann jedoch auch auf einer oder beiden Seiten der Membran jeweils mittels eines Fluidkanals mit dem Herzinneren einerseits und dem Perikardium oder dem Thoraxbereich andererseits verbunden sein, so dass auf das Differenzdruckmesselement, insbesondere die Membran, die beiden zu vergleichenden Drücke wirken. Die entsprechenden Fluidkanäle können durch feste rohrartige Elemente oder Kanülen oder Katheter gebildet sein. Sie können auch durch Bohrungen und/oder Kanäle innerhalb eines festen Körpers, beispielsweise eines Gehäuses eines Geräts, gebildet sein, beispielsweise in oder an einer Herzpumpe.

Eine weitere Ausgestaltung der Lösung kann beispielsweise auch vorsehen, dass der Differenzdrucksensor einerseits mit einem Bereich innerhalb der Pumpe oder in ihrem Ansaugbereich oder ihrem Ausstoßbereich und andererseits mit einem Bereich außerhalb des Pumpengehäuses verbunden ist. Eine solche Messung führt grundsätzlich dazu, dass ein Differenzdruck zwischen dem Inneren der Pumpe, entweder im Ansaug- oder im Ausstoßbereich, einerseits und außerhalb der Pumpe im Thorax andererseits gemessen wird. Die durch den Betrieb der Pumpe geschaffene Druckdifferenz zwischen Ansaug- und Ausstoßbereich kann bei der Bestimmung des Drucks in den Blutgefäßen und im Herzen berücksichtigt werden. Somit ergibt sich eine besonders günstige Anordnung aller Kanäle an einer solchen Pumpe oder im Bereich der Pumpe in ihrem Inneren und auf ihrer Außenseite, so dass die Gesamtanordnung, gebildet aus der Sensoreinrichtung und einer derartigen Herzpumpe, besonders günstig herzustellen, zu implantieren und zu betreiben ist.

Eine weitere Ausgestaltung der Lösung kann vorsehen, dass der Kanal durch den Innenraum eines Pumpengehäuses verläuft und wenigstens teilweise, insbesondere abschnittsweise, allseitig von dem durch die Pumpe geförderten Blut umgeben ist. Wenn ein solcher Kanal im Inneren einer Pumpe verläuft, ist die Anordnung besonders platzsparend und wenig anfällig. Es sind dabei jedoch Probleme des Platzbedarfs innerhalb des Pumpengehäuses zu lösen, weil dort beispielsweise bewegte Teile Raum benötigen und durch den Kanal nicht behindert werden dürfen. Besondere Probleme können sich beispielsweise bei der Verwendung von axial fördernden Rotorpumpen ergeben, da bei solchen Pumpen der größte Teil des Innenraums des Pumpengehäuses bei der Rotation des Förderrotors überstrichen wird.

Gute Lösungsmöglichkeiten ergeben sich beispielsweise bei der Verwendung solcher Rotoren, die in ihrem Rotationsachsenbereich oder im Inneren einer Nabe einen Hohlraum aufweisen, in dem ein solcher Fluidkanal angeordnet werden kann. Eine derartige Pumpe kann beispielsweise auch in Axialrichtung Blut ansaugen und dieses in Radialrichtung fördern. Andererseits kann auch in oder an einer Pumpe ein derartiger Fluidkanal an der Gehäusewand getrennt vom Pumpeninnenraum verlaufen oder als Bohrung in die Wand des Pumpengehäuses eingebracht sein.

Die Lösung kann auch dadurch ausgestaltet sein, dass die Sensoreinrichtung zwei Absolutdrucksensoren aufweist, von denen der erste im Inneren des Herzens angeordnet oder mit dem Inneren des Herzens über einen Fluidkanal verbunden ist und der zweite außerhalb des Herzens im Thorax angeordnet oder mit einem Bereich außerhalb des Herzens im Thorax über einen Fluidkanal verbunden ist und die mit einer Einrichtung zur Druckdifferenzermittlung verbunden sind. In diesem Fall kann je nach Anordnung des Geräts, insbesondere der Herzpumpe, wenigstens einer der Absolutdrucksensoren am Gehäuse des Geräts befestigt sein und den Druck außerhalb oder innerhalb des Gehäuses messen. Bei einer Herzpumpe kann beispielsweise bei einem außerhalb des Herzens liegenden Teil des Pumpengehäuses ein Absolutdrucksensor auf der Außenseite des Gehäuses angeordnet und auch gegebenenfalls an diesem befestigt sein und mit dem Perikardium oder dem Thorax außerhalb des Perikardiums unmittelbar oder mittels eines Fluidkanals kommunizieren. Ein derartiger Absolutdrucksensor kann auch im Inneren des Pumpengehäuses angeordnet sein und mittels eines durch die Wand des Pumpengehäuses verlaufenden Kanals mit den entsprechenden Bereichen außerhalb des Herzens kommunizieren. Ein Absolutdrucksensor kann auch im Inneren des Herzens an der Außen- oder Innenseite des Pumpengehäuses fest angeordnet und mit dem Gehäuse verbunden sein.

Es kann dann vorgesehen sein, dass die Einrichtung zur Druckdifferenzermittlung außerhalb des Herzens im Thorax oder außerhalb des Patientenkörpers angeordnet ist. Zudem kann vorgesehen sein, dass die Sensoren mit einer Funkeinrichtung zur Übermittlung von Messwerten oder mit einer elektrischen, die Herzwand durchsetzenden Leitung verbunden sind. Die Sensoren können auch als passive, funkabfragbare Oberflächenwellensensoren in Form eines Transponders ausgebildet sein.

Da die Druckdifferenz zwischen den ermittelten Messwerten beider Drucksensoren zu ermitteln ist, können diese Messwerte zunächst an eine Einrichtung zur Druckdifferenzermittlung übermittelt werden. Die Übermittlung der Werte kann dabei elektrisch oder mittels einer Funkübermittlung oder anderer nicht körperlich gebundener Übermittlungsverfahren geschehen. Die Druckdifferenzermittlungseinrichtung kann dann in analoger oder digitaler Weise eine Differenzbildung durchführen und den Druckdifferenzwert zur Verfügung stellen oder direkt an eine Steuereinrichtung des Geräts, insbesondere der Herzpumpe, übermitteln. Die Steuereinrichtung des Geräts kann auch unmittelbar die Druckdifferenzermittlung mit übernehmen.

Bei der Verwendung eines einzelnen Differenzdrucksensors, auf den beide zu vergleichenden Drücke wirken, ist eine derartige Differenzbildung selbstverständlich nicht notwendig, bzw. sie geschieht unmittelbar durch Anwendung des entsprechenden Differenzdrucksensorprinzips.

Die Lösung der Aufgabe bezieht sich weiter auch auf ein Verfahren zur Steuerung oder Regelung eines Aggregats in einem Patientenkörper unter Berücksichtigung eines im Herzen des Patienten erfassten Drucks, wobei der Druck als Differenzdruck zwischen dem Inneren des Herzens und dem Bereich außerhalb des Herzens im Thorax erfasst und der Steuerung oder Regelung zugrunde gelegt wird.

Zur Durchführung des Verfahrens kann die oben beschriebene Vorrichtung, insbesondere in Verbindung mit dem Steuergerät eines implantierten Geräts, wie beispielsweise einer Herzpumpe, eines Herzschrittmachers oder eines Defibrillators, verwendet werden.

Zudem bezieht sich die Lösung auf ein Computerprogrammprodukt mit einem Computerprogramm zur Steuerung oder Regelung eines Aggregates in einem Patientenkörper unter Berücksichtigung eines im Herzen des Patienten erfassten Drucks, wobei der Druck als Differenzdruck zwischen dem Inneren des Herzens und dem Bereich außerhalb des Herzens im Thorax ermittelt und im Computerprogramm als Parameter verwendet wird. Ein derartiges Computerprogramm wird zum Betreiben beispielsweise eines Mikrocontrollers verwendet, der eine Verarbeitung der erfassten Messdaten leistet. Das Programm kann jedoch auch als Teilprogramm oder Programmmodul beim Betrieb eines Steuergeräts eines entsprechenden Aggregats, wie beispielsweise einer Herzpumpe, betrieben werden.

Zur Lösung der Aufgabe kann zudem eine Regeleinrichtung zur Regelung einer Herzpumpe vorgesehen sein, bei der Parameter wie z.B. Füllungsdrücke des Herzens basierend auf der Druckdifferenz zwischen dem Druck im Inneren des Herzens und außerhalb des Herzens im Thorax, ermittelt durch eine Vorrichtung nach einem der Ansprüche 1 bis 11, verwendet werden und bei der die Förderleistung der Pumpe als Stellgröße dient.

Außerdem kann die Lösung in Verbindung mit einem Herzschrittmacher auch eine Regeleinrichtung zur Regelung eines Herzschrittmachers vorsehen, bei der Parameter wie z.B. Füllungsdrücke des Herzens basierend auf der Druckdifferenz zwischen dem Herzinneren und dem Raum außerhalb des Herzens im Thorax, ermittelt durch eine Vorrichtung nach einem der Ansprüche 1 bis 11, verwendet werden und bei der als Zielgröße der Regelung die Förderleistung des Herzens dient.

Im Folgenden werden die angegebenen Lösungen der Aufgabe in Figuren einer Zeichnung gezeigt und nachfolgend erläutert. Dabei zeigt
- Fig. 1: einen Patientenkörper in schematischer Darstellung mit der Darstellung eines Herzens und einer Herzpumpe,
- Fig. 2: eine schematische Darstellung eines Herzens als Hohlraum mit einer an dieses angeschlossenen Herzpumpe mit Sensoren,
- Fig. 3: schematisch die Darstellung eines Herzens mit zwei Sensoren, die mit einem Gerät verbunden sind,
- Fig. 4: die schematische Darstellung eines Herzens mit zwei möglichen Anordnungen von Geräten, die jeweils Sensoren zur Druckmessung aufweisen,
- Fig. 5: den Aufbau einer Axial/Radialpumpe mit einem Differenzdrucksensor sowie
- Fig. 6: den Aufbau einer Axialpumpe mit einem Differenzdrucksensor.

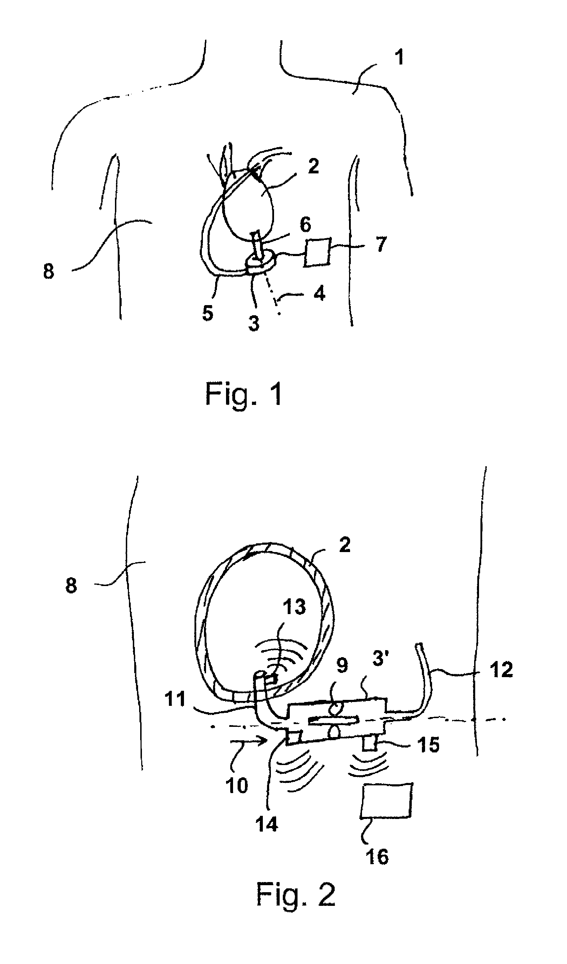

Figur 1 zeigt schematisch den Körper 1 eines Patienten, wobei gesondert und schematisch das Herz 2 mit einer implantierten Herzpumpe 3 dargestellt ist.

Die Herzpumpe 3 weist einen Rotor auf, der um die Achse 4 drehbar ist und dabei Blut in einen Katheter 5 fördert. Die Pumpe 3 saugt dabei durch einen Ansaugstutzen 6, der eine Wand des Herzens 2 durchsetzt, Blut aus dem Inneren des Herzens, beispielsweise aus einem Atrium oder einem Ventrikel, an und fördert dieses über die Kanüle / den Katheter 5 in ein Blutgefäß.

Es ist zudem ein Steuergerät 7 dargestellt, das die Pumpe 3, beispielsweise mittels elektrischer Signale, ansteuert. Die Pumpe 3 wird durch einen Elektromotor mit Wicklungen und Magneten angetrieben. Dabei kann es sich bei dem Motor um einen bürstenlosen, beispielsweise im Pulsweitenmodulationsverfahren angesteuerten Elektromotor handeln.

Der Rotor der Pumpe 3 kann als Radialrotor ausgebildet sein, der axial einströmendes Fluid in radialer Richtung beschleunigt. In einem radial außen liegenden Sammelraum wird dann das geförderte Fluid/Blut gesammelt und durch den erzeugten Druck radial außen in die Kanüle 5 geleitet.

Zur Steuerung der Pumpe 3 mittels des Steuergeräts 7 ist die Berücksichtigung der Druckverhältnisse im Herzen 2 im Vergleich zu dem im Thorax 8 außerhalb des Herzens herrschenden Druck wichtig. Der Differenzdruck zwischen dem Raum innerhalb des Herzens 2 und dem Thorax 8 wird durch eine Sensoreinrichtung gemessen, auf die weiter unten noch genauer eingegangen wird.

Figur 2 zeigt schematisch in einem Querschnitt das Herz 2 des Patienten, das hier nur als Hohlraum dargestellt ist und sich im Thorax 8 befindet. Es ist eine Herzpumpe 3' vorgesehen, die als axial fördernde Pumpe mit einem Rotor 9 ausgebildet ist. Die Pumpe fördert beispielsweise Blut aus dem Inneren des Herzens 2 in Richtung des Pfeils 10, saugt dieses somit durch die Ansaugkanüle 11 aus dem Herzen an und stößt es durch die Ausflusskanüle 12 in Richtung eines nicht dargestellten Blutgefäßes aus. Zur Messung der Druckdifferenz zwischen dem Inneren des Herzens 2 und dem Thorax können wenigstens zwei Sensoren einer Sensoreinrichtung vorgesehen sein. In Figur 2 sind insgesamt drei Sensoren dargestellt, wobei die Sensoren 13, 14 als Alternativen gedacht sind und jeweils den Druck im Herzinneren messen. Dabei ist der Sensor 13 unmittelbar im Herzinneren angeordnet, während der Sensor 14 im Inneren der Pumpe durch den Ansaugstutzen 11 mit dem Inneren des Herzens verbunden ist und, sofern von Strömungswiderständen abgesehen wird, unmittelbar den Druck im Herzinneren misst. Der Sensor 15 ist dagegen außerhalb der Pumpe 3' im Thorax 8 angeordnet und misst den dortigen Druck.

Die drei Sensoren 13, 14, 15 sind jeweils mit Funkeinrichtungen oder Induktionsspulen ausgestattet, die ihnen erlauben, erfasste Messwerte an eine Einrichtung 16 zur Bestimmung der Druckdifferenz zu senden. Die Einrichtung 16 kann beispielsweise als Mikrocontroller ausgebildet sein und mit einem Empfänger zum Empfangen der gemessenen Daten ausgestattet sein. Ein Computerprogramm, das auf dem Mikrocontroller läuft, ermittelt die Druckdifferenz, die durch die verschiedenen Sensoren 13, 14, 15 der Sensoreinrichtung erfasst wird. Diese kann dann an eine Steuereinrichtung der Pumpe 3' weitergeleitet werden. Die Sensoren 13, 14, 15 sind unmittelbar an der Herzpumpe 3' bzw. am Ansaugstutzen 11 der Pumpe angeordnet und befestigt. Sie können dabei, wie beispielsweise der Sensor 15, an der Außenseite des Pumpengehäuses befestigt sein, jedoch auch, wie der Sensor 14, im Inneren des Pumpenraums auf der Innenseite des Gehäuses.

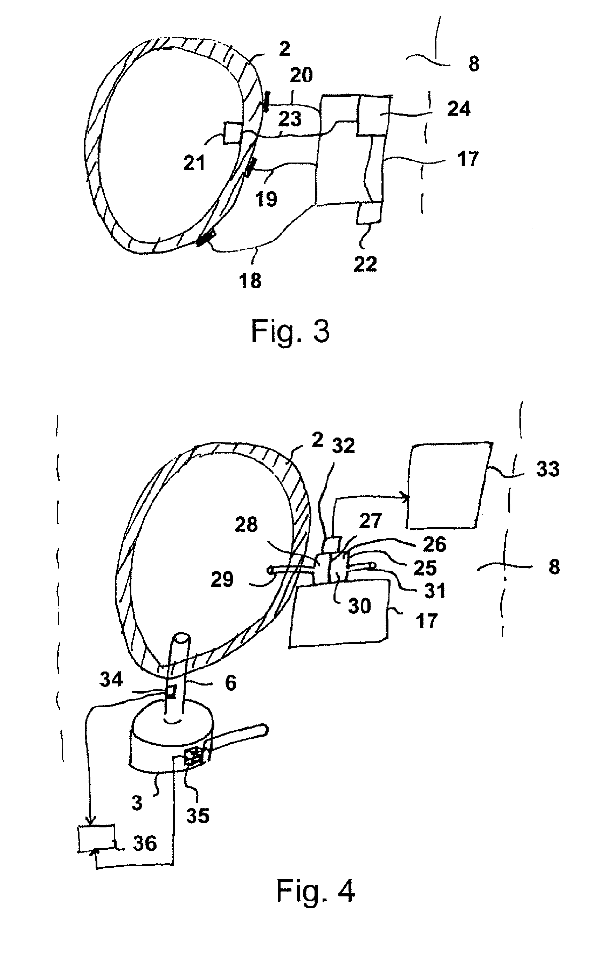

In Figur 3 ist ein Herzschrittmacher 17 dargestellt, der im Thorax 8 eines Patienten implantiert ist. Der Herzschrittmacher 17 ist mittels verschiedener Leitungen 18, 19, 20 mit Elektroden verbunden, die unmittelbar an der Herzwand des Herzens 2 anliegen. Zur Berücksichtigung des transmuralen Drucks, d. h. des Differenzdrucks zwischen dem Inneren des Herzens 2 und dem Thorax 8, sind zwei Sensoren 21, 22 vorgesehen, wobei der erste Sensor 21 im Inneren des Herzens 2 angeordnet und mittels einer elektrischen Leitung 23 mit dem Herzschrittmacher 17 verbunden ist. Der Sensor 22 ist unmittelbar am Gehäuse am Herzschrittmacher 17 außen befestigt und dient der Messung der Drucks im Thorax. Beide Sensoren 21, 22 sind mittels Leitungen mit einer Differenzdruckermittlungseinrichtung 24 verbunden, die in den Herzschrittmacher 17 integriert ist. Der ermittelte Differenzdruck kann damit innerhalb verschiedener Algorithmen des Herzschrittmachers 17 verwendet werden.

Figur 4 zeigt schematisch das Herz 2 eines Patienten mit zwei alternativ verwendbaren Aggregaten, die jeweils für sich eine Differenzdruckermittlung zulassen. Im rechten Teil der Figur ist ein Herzschrittmacher 17 nur schematisch als Box eingezeichnet. An diesem befestigt ist ein Differenzdrucksensor 25, der in einem Messraum 26 eine Trennmembran 27 vorsieht. Der erste Teilraum 28 auf der dem Herzen 2 zugewandten Seite der Membran 27 ist über eine Kanüle 29 mit dem Inneren des Herzens verbunden. Der zweite Teilraum 30, der auf der dem Herzen 2 abgewandten Seite der Membran 27 liegt, ist mittels einer Kanüle 31 mit dem Inneren des Thorax 8 verbunden. Die Membranstellung der elastischen, auslenkbaren Membran 27 zeigt unmittelbar die Druckdifferenz zwischen dem Inneren des Herzens 2 und dem Thorax an. Diese wird durch einen Sensor und Mikrocontroller 32 erfasst und ausgewertet und als Wert der Druckdifferenz unmittelbar der Steuereinheit 33 zugeführt. Diese kann ihrerseits auf den Herzschrittmacher 17 wirken und mit diesem in Verbindung stehen.

In Figur 4 ist zudem eine Herzpumpe 3 eingezeichnet, die als Axial/Radialpumpe ausgebildet ist und einen Ansaugstutzen 6 aufweist, der durch die Herzwand des Herzens 2 hindurchragt und dicht in die Öffnung der Herzwand eingepasst ist. Innerhalb des Ansaugstutzens 6 ist ein erster Sensor 34 angeordnet, der über das Innere des Ansaugstutzens 6 mit dem Inneren des Herzens 2 in Verbindung steht und den dort herrschenden Absolutdruck misst. Zudem ist an der Außenseite der Pumpe 3 ein Sensor 35 angeordnet, der den Absolutdruck in der Umgebung der Pumpe 3, also im Thorax, erfasst. Beide Sensoren 34, 35 sind über Funk oder über eine elektrische Leitung mit einer Differenzdruckermittlungseinrichtung 36 verbunden, die den Differenzdruck zwischen Herzinnerem und Thorax ermittelt.

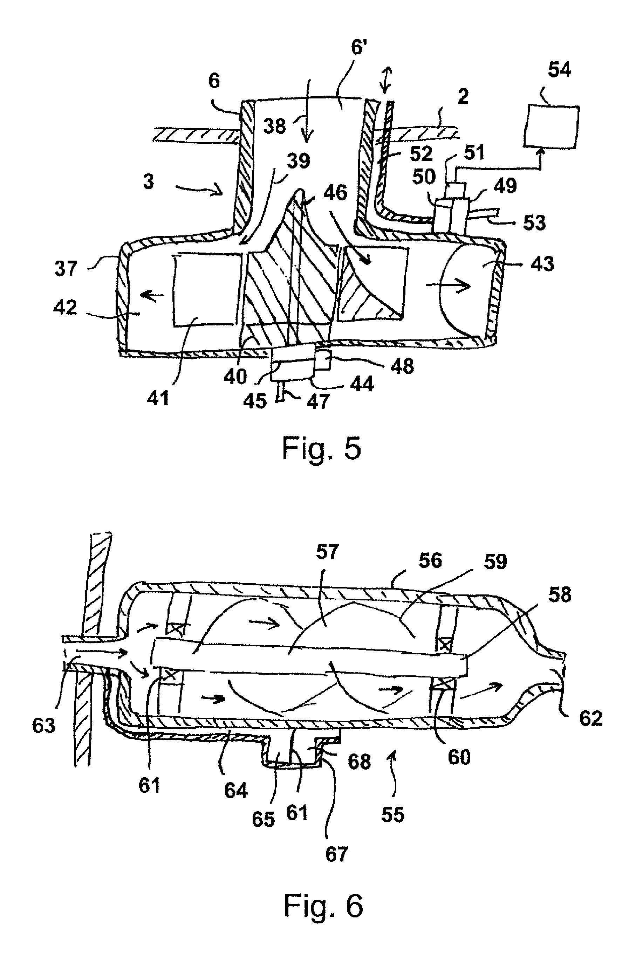

In Figur 5 ist eine Axial/Radialpumpe 3 beispielhaft detaillierter gezeigt. Die Pumpe weist ein Gehäuse 37 mit einem Ansaugstutzen 6 auf, der die Wand des Herzens 2 durchsetzt. In dem Ansaugstutzen 6 ist ein Einströmkanal/Ansaugkanal 6' gebildet, durch den Blut in Richtung der Pfeile 38, 39 angesaugt wird.

Der Pumpenmotor weist einen innenliegenden Stator 40 auf, auf dem der Rotor 41 hydrostatisch und/oder magnetisch sowie in Radial- als auch in Axialrichtung gelagert sein kann. Der Pumpenmotor kann als bürstenloser Elektromotor ausgebildet sein. Im Rotor sind Fließkanäle für das Blut integriert, die dieses in axialer Richtung einfließen lassen und durch Zentrifugalwirkung in radialer Richtung ausstoßen. Um den Rotor 41 herum ist ein ringförmiger Sammelraum 42 in dem Gehäuse 37 gebildet, in dem das Blut sich sammelt und in Umfangsrichtung bis zu einer Ausströmöffnung 43 fließt, von wo es in eine Ausströmkanüle weiterbewegt wird.

Es sind anhand der Pumpe der Figur 5 zwei Beispiele für eine Differenzdruckmessung dargestellt. Zunächst ist ein Differenzdrucksensor 44 mit einer Membran 45 dargestellt, wobei der Sensor einerseits über einen Kanal 46 im Stator mit dem Ansaugbereich der Pumpe im Ansaugstutzen und andererseits mit dem Pumpenäußeren über einen Fluidkanal 47 kommuniziert. Der Differenzdruck wird in einer Auswerteeinrichtung 48 ausgewertet und entsprechend an ein Aggregat weitergeleitet.

Die zweite Differenzdruckeinrichtung, die alternativ an einer solchen Pumpe denkbar ist, weist einen Differenzdrucksensor 49 mit einer Membran 50 sowie eine Auswerteeinrichtung 51 auf. Der Sensor 49 ist auf einer Seite der Membran 50 über einen Kanal 52 mit dem Herzinneren verbunden, der im oder am Gehäuse 37 parallel zu dem Ansaugkanal 6' verläuft. Der Kanal 52 kann durch ein außen auf das Gehäuse 37 aufgesetztes gebogenes Rohr oder durch eine Bohrung in der Wand des Gehäuses 37 oder auch durch ein an der Innenwand des Gehäuses 37 verlaufendes Rohr gebildet sein.

Der Sensor 49 kommuniziert auf der anderen Seite der Membran über eine Kanüle 53 mit der äußeren Umgebung des Pumpengehäuses 37 im Thorax. Die Differenzdruckermittlungseinrichtung 51 kommuniziert mit einem Aggregat 54.

In Figur 6 ist eine Axialpumpe 55 mit einem teilweise zylindrischen Gehäuse 56 dargestellt, in dem ein Rotor 57 drehbar gelagert ist. Der Rotor 57 weist eine Nabe 58 mit Förderelementen 59 auf. Die Nabe 58 ist in zwei Lagern 60, 61 drehbar gelagert und üblicherweise mittels eines Motors oder einer flexiblen Welle angetrieben, die in der Figur nicht dargestellt ist. Die flexible Welle ragt üblicherweise durch die Abströmöffnung 62 des Gehäuses 56 heraus. Die Einströmöffnung ist mit 63 bezeichnet.

Mit der Einströmöffnung 63 ist ein Kanal 64 verbunden, der außen an der Wand des Gehäuses 56 verläuft und zu einem ersten Teilraum 65 auf einer Seite der Membran 66 eines Differenzdrucksensors 67 führt. Der zweite Teilraum 68 des Differenzdrucksensors 67 ist über eine Öffnung und/oder einen Fluidkanal mit der äußeren Umgebung des Pumpengehäuses 56 verbunden. Der Fluidkanal 64 kann auch innerhalb der Gehäusewand des Gehäuses 56 oder an der Innenseite der Gehäusewand des Gehäuses 56 zwischen der Einströmöffnung 63 und dem Differenzdrucksensor 67 verlaufen.

Durch die beschriebenen Lösungen ist in einfacher Weise die Bestimmung des Differenzdrucks zwischen dem absoluten Druck im Herzen des Patienten und dem Thorax möglich, wobei durch die Integration der Sensoreinrichtung wenigstens teilweise in ein implantierbares Aggregat sowohl die Herstellung als auch die Implantierung erleichtert wird.

Mit der beschriebenen Sensoreinrichtung kann der wirkliche Füllungsdruck des Herzens ohne mögliche Einflüsse von Atmung, "Pressen" oder pathologischen Veränderungen des Thoraxdrucks ermittelt werden. Daher kann mit der gleichen Anzahl von Drucksensoren wie bisher ein vorteilhafteres Signal gemessen werden. Das ermöglicht eine schnellere Regelung von Herzpumpen als bisher, die robuster gegen diese Störeinflüsse ist. Dies würde zu einer konsequenten Ansaugprävention und durch die schnellere Adaption auf körperliche Belastung zu einer verbesserten Lebensqualität der Patienten führen. Auch eine Detektion einer pathologischen Veränderung im Thoraxdruck (z.B. Perikarderguss, Herzbeuteltamponade) wäre denkbar.

## Patentansprüche

1. Vorrichtung zur Druckmessung am Herzen (2) eines Patienten, **gekennzeichnet durch** eine Sensoreinrichtung (13, 14, 15, 21, 22, 25, 27, 32, 34, 35, 44, 45, 48, 49, 50, 51, 67), die die Druckdifferenz zwischen einer Stelle innerhalb des Herzens einerseits und dem außerhalb des Herzens innerhalb des Thorax (8) liegenden Raum andererseits erfasst.

2. Vorrichtung nach Anspruch 1, **dadurch gekennzeichnet, dass** die Sensoreinrichtung einen die Herzaußenwand durchsetzenden Kanal (6, 11, 29, 46, 52, 64) aufweist, der sowohl mit einem außerhalb des Herzens (2) im Thorax (8) liegenden Bereich als auch mit einem innerhalb des Herzens (2) liegenden Bereich kommuniziert, und dass ein Differenzdrucksensor (25, 27, 32, 44, 45, 48, 49, 50, 51, 67) sowohl mit dem ersten, außerhalb des Herzens liegenden als auch mit dem zweiten, innerhalb des Herzens liegenden Bereich verbunden ist.

3. Vorrichtung nach Anspruch 2, **dadurch gekennzeichnet, dass** der Differenzdrucksensor (25, 27, 32, 44, 45, 48, 49, 50, 51, 67) eine Vorrichtung (6) aufweist, die eine durchgehende Öffnung in der Herzwand verschließt.

4. Vorrichtung nach Anspruch 2, **dadurch gekennzeichnet, dass** der Kanal (6,11, 29, 46, 52, 64) in oder an einer Blutpumpe (3, 3', 55) gebildet ist.

5. Vorrichtung nach Anspruch 4, **dadurch gekennzeichnet, dass** der Differenzdrucksensor (25, 27, 32, 44, 45, 48, 49, 50, 51, 67) einerseits mit einem Bereich innerhalb der Pumpe (3, 3', 55) oder in ihrem Ansaugbereich oder ihrem Ausstoßbereich und andererseits mit einem Bereich außerhalb des Pumpengehäuses (37, 56) und der Blutgefäße verbunden ist.

6. Vorrichtung nach Anspruch 2 oder einem der folgenden, **dadurch gekennzeichnet, dass** der Kanal (46) durch den Innenraum eines Pumpengehäuses (37, 56) verläuft und wenigstens teilweise, insbesondere abschnittsweise, allseitig von dem durch die Pumpe (3, 3', 55) geförderten Blut umgeben ist, wobei insbesondere vorgesehen ist, dass die Pumpe in Axialrichtung Blut ansaugt und dies in Radialrichtung fördert.

7. Vorrichtung nach Anspruch 2 oder einem der folgenden, **gekennzeichnet durch** eine Pumpe (3, 3', 55) mit einem an ihrer Gehäusewand (37, 56) getrennt vom Pumpenraum verlaufenden Kanal (52, 64).

8. Vorrichtung nach Anspruch 1, **dadurch gekennzeichnet, dass** die Sensoreinrichtung zwei Absolutdrucksensoren (13, 14, 15, 21, 22) aufweist, von denen der erste (13, 14, 21) im Inneren des Herzens (2) angeordnet oder mit dem Inneren des Herzens (2) über einen Fluidkanal verbunden ist und der zweite (15, 22) außerhalb des Herzens (2) im Thorax (8) angeordnet oder mit einem Bereich außerhalb des Herzens im Thorax (8) über einen Fluidkanal verbunden ist und die mit einer Einrichtung zur Druckdifferenzermittlung verbunden sind.

9. Vorrichtung nach Anspruch 8, **dadurch gekennzeichnet, dass** die Einrichtung zur Druckdifferenzermittlung (24, 32) außerhalb des Herzens (2) im Thorax (8) oder außerhalb des Patientenkörpers angeordnet ist.

10. Vorrichtung nach Anspruch 8 oder 9, **dadurch gekennzeichnet, dass** der erste Sensor mit einer Funkeinrichtung zur Übermittlung von Messwerten oder mit einer elektrischen, die Herzwand durchsetzenden Leitung verbunden ist.

11. Vorrichtung nach einem der vorangehenden Ansprüche, **dadurch gekennzeichnet, dass** wenigstens ein Sensor der Sensoreinrichtung als Oberflächenwellenfilter ausgebildet ist und/oder dass Induktionsspulen zur Übermittlung von Sensordaten und/oder der Energieversorgung eines Sensors vorgesehen sind.

12. Verfahren zur Steuerung oder Regelung eines Aggregates in einem Patientenkörper unter Berücksichtigung eines im Herzen (2) des Patienten erfassten Drucks, **dadurch gekennzeichnet, dass** der Druck als Differenzdruck zwischen dem Inneren des Herzens und dem Bereich außerhalb des Herzens im Thorax (8) erfasst und der Steuerung oder Regelung zugrunde gelegt wird.

13. Computerprogrammprodukt mit einem Computerprogramm zur Steuerung oder Regelung eines Aggregates (3, 3', 17, 55) in einem Patientenkörper unter Berücksichtigung eines im Herzen (2) des Patienten erfassten Drucks, wobei der Druck als Differenzdruck zwischen dem Inneren des Herzens (2) und dem Bereich außerhalb des Herzens im Thorax (8) ermittelt und im Computerprogramm als Parameter verwendet wird.

14. Regeleinrichtung zur Regelung einer Herzpumpe (3, 3', 55), bei der als Messgröße die Druckdifferenz zwischen dem Druck im Inneren des Herzens (2) und außerhalb des Herzens (2) im Thorax (8), ermittelt durch eine Vorrichtung nach einem der Ansprüche 1 bis 11, verwendet wird und bei der die Förderleistung der Pumpe (3, 3', 55) als Stellgröße dient.

15. Regeleinrichtung zur Regelung eines Herzschrittmachers (17), bei der als Messgröße die Druckdifferenz zwischen dem Herzinneren und dem Raum außerhalb des Herzens (2) im Thorax (8), ermittelt durch eine Vorrichtung nach einem der Ansprüche 1 bis 11, verwendet wird und bei der als Zielgröße der Regelung die Förderleistung des Herzens (2) dient.
